# EUROPEAN PATENT APPLICATION

(11) **EP 4 403 623 A1**
(43) Date of publication of application: **24.07.2024**
(21) Application number: 22870149.6
(22) Date of filing: 09.08.2022
(51) Int. Cl.: C12N 1/12, A01N 65/03, C05F 11/08, A01P 21/00, C12R 1/89

(54) **MICROALGAE STRAIN HAVING EFFECT OF PROMOTING PLANT GROWTH AND USE THEREOF**

(30) Priority: 17.09.2021 KR 20210125207
(71) Applicant: CJ Cheiljedang Corporation, Seoul 04560 (KR)
(72) Inventor: SHIN, Won Sub, Seoul 04560 (KR); CHOI, Jung-Woon, Seoul 04560 (KR); JANG, Sunghoon, Seoul 04560 (KR); KANG, Yuna, Seoul 04560 (KR); KANG, Hae-Won, Seoul 04560 (KR); OH, Youngjoo, Seoul 04560 (KR); KIM, Gyuree, Seoul 04560 (KR); KIM, Ji Young, Seoul 04560 (KR)
(74) Representative: Bates, Philip Ian
(86) International application number: PCT/KR2022/011885
(87) International publication number: WO 2023/043063

(57) **Abstract**

The present application relates to a novel microalgae strain with the efficacy of promoting plant growth and a use thereof. When cultured in the presence of fermentation exhaust gas, a novel Chlorella vulgaris CD02-3002 strain according to an aspect grows fast with a high efficiency of photosynthesis and thus can effectively reduce the exhaust gas generated by microbial fermentation. Having the effect of promoting plant growth when applied to plants, a culture of the strain or a supernatant of the culture can be used as a fertilizer for plants and thus can be advantageously applied as a novel carbon reduction technique.

## Description

### [TECHNICAL FIELD]

### Cross-Reference to Related Application(s)

The present application claims the priority based on Korean Patent Application No. 10-2021-0125207 filed on September 17, 2021, and the entire contents disclosed in the documents of the corresponding Korean Patent Application are incorporated by reference as a part of the present description.

The present application relates to a novel microalgae strain having an effect of promoting plant growth and use thereof.

### [BACKGROUND ART]

As the scale of the bio-industry expands, various products can be produced based on the fermentation technology of microorganisms, and in particular, with the recent growth of White Bio field, the microbial fermentation industry is also growing. However, since fermentation is basically a process that emits carbon dioxide, as the industry in the microbial fermentation field develops, there is a problem that the emission of greenhouse gas generated by fermentation is greatly increased. In addition, as climate change issues are emerging around the world, efforts to reduce greenhouse gas, which is pointed out as a main cause of climate change, are being carried out from various angles, and thus, reducing greenhouse gas generated by microbial fermentation is also becoming a major issue.

Until now, technologies that can contribute to carbon reduction by mainly suppressing the emission of greenhouse gas itself have been commercialized, but it is considered very difficult to reduce the emission of carbon dioxide in the microbial fermentation process. In addition, technology development that can directly capture and reduce greenhouse gas is still at a minimal level, and in particular, since carbon dioxide exists in a very low concentration in the atmosphere and is a stable material with very low reactivity, there is a difficulty in developing a technology for reducing already emitted carbon dioxide. In this regard, as a kind of biological carbon reduction technologies, a method of using a photosynthetic organism may be considered, but there is a problem in that it is difficult to apply to actual industry because the profit structure other than the purpose of carbon reduction is unclear. Accordingly, there is a need to develop an industrially applicable carbon reduction technology while being able to reduce carbon dioxide generated by microbial fermentation in an environmentally friendly way.

### [DISCLOSURE]

### [TECHNICAL PROBLEM]

An object of the present invention is to provide a *Chlorella vulgaris* CD02-3002 strain having an effect of promoting plant growth, deposited with Accession number KCTC14659BP.

Another object of the present invention is to provide a composition for promoting plant growth, comprising any one or more selected from the group consisting of a *Chlorella vulgaris* CD02-3002 strain having an effect of promoting plant growth, deposited with Accession number KCTC14659BP; culture or culture supernatant of the strain; concentrate of the culture or culture supernatant; a dried matter of the culture, culture supernatant or concentrate; and lysate of the dried matter.

Other object of the present invention is to provide a fertilizer for plants comprising the composition for promoting plant growth.

Other object of the present invention is to provide a method of culturing a *Chlorella vulgaris* CD02-3002 strain, comprising culturing a *Chlorella vulgaris* CD02-3002 strain having an effect of promoting plant growth, deposited with Accession number KCTC14659BP in the presence of fermentation exhaust gas.

Other object of the present invention is to provide a method of promoting plant growth, comprising preparing a composition for promoting plant growth comprising any one or more selected from the group consisting of a *Chlorella vulgaris* CD02-3002 strain having an effect of promoting plant growth, deposited with Accession number KCTC14659BP; culture or culture supernatant of the strain; concentrate of the culture or culture supernatant; a dried matter of the culture, culture supernatant or concentrate; and lysate of the dried matter; and contacting the composition for promoting plant growth to a plant.

Other object of the present invention is to provide a use for manufacturing a fertilizer for plants or a use for promoting plant growth, of a *Chlorella vulgaris* CD02-3002 strain having an effect of promoting plant growth, deposited with Accession number KCTC14659BP.

Other object of the present invention is to provide a use for manufacturing a fertilizer for plants or a use for promoting plant growth, of a composition, comprising any one or more selected from the group consisting of a *Chlorella vulgaris* CD02-3002 strain having an effect of promoting plant growth, deposited with Accession number KCTC14659BP; culture or culture supernatant of the strain; concentrate of the culture or culture supernatant; a dried matter of the culture, culture supernatant or concentrate; and lysate of the dried matter.

### [TECHNICAL SOLUTION]

Each description and embodiment disclosed in the present application can be applied to each other description and embodiment. In other words, all combinations of various elements disclosed in the present application fall within the scope of the present invention. In addition, it cannot be seen that the scope of the present invention is limited by the specific description described below. Furthermore, those skilled in the art can recognize or confirm many equivalents to specific aspect of the present invention described in the present application using only common experiments. Moreover, such equivalents are intended to be comprised in the present invention.

One embodiment of the present invention provides a *Chlorella vulgaris* CD02-3002 strain having an effect of promoting plant growth, deposited with Accession number KCTC14659BP.

The plant may be a plant of the genus *Arabidopsis.* For example, the plant of the genus *Arabidopsis* may be *Arabidopsis thaliana,* but not limited thereto.

The term used in the present description, "plant growth" means a process in which a plant seed germinates to give out roots, stems and leaves, or to gradually increase in size and weight thereafter, and the term "promoting plant growth" means promoting germination of a plant seed or an increase in size and/or weight of the germinated plant.

The *Chlorella vulgaris* CD02-3002 strain may have the 18S rRNA nucleotide sequence of SEQ ID NO: 1, but not limited thereto. For example, the *Chlorella vulgaris* CD02-3002 strain may have 18S rRNA consisting of a nucleotide sequence having sequence identity of 80% or more, 85% or more, 90% or more, 95% or more, 98% or more, or 99% or more with the nucleotide sequence of SEQ ID NO: 1, but not limited thereto.

Another aspect of the present invention provides a composition for promoting plant growth, comprising any one or more selected from the group consisting of a *Chlorella vulgaris* CD02-3002 strain having an effect of promoting plant growth, deposited with Accession number KCTC14659BP; culture or culture supernatant of the strain; concentrate of the culture or culture supernatant; a dried matter of the culture, culture supernatant or concentrate; and lysate of the dried matter.

The *Chlorella vulgaris* CD02-3002 strain is as described above.

The "culture" of the strain refers to a product produced by culturing the strain, and specifically may be a culture solution comprising the strain, and the "culture supernatant" may be culture filtrate in which the strain is removed from the culture or culture solution of the strain. The "concentrate" may mean concentrate of the culture or culture supernatant. The "dried matter" is the culture, culture supernatant of the strain or concentrate thereof from which moisture is removed, and for example, it may be in a form of a dried microbial cell of the strain, but not limited thereto. In addition, the "lysate" of the dried matter is a generic term for the result of lysing the dried matter in which moisture is removed, and for example, may be a dried microbial cell powder of the strain, but not limited thereto. The culture of the strain may be prepared according to a culturing method known in the art by inoculating the strain in a culture medium, or may be prepared by a method comprising culturing the strain in the presence of fermentation exhaust gas. The concentrate, dried matter thereof or lysate thereof may be prepared according to a treatment or drying method of microalgae known in the art or culture thereof.

The plant may be a plant of the genus *Arabidopsis.* For example, the plant of the genus *Arabidopsis* may be *Arabidopsis thaliana,* but not limited thereto.

The composition for promoting plant growth may contain a preservative, stabilizer, wetting agent or emulsifier, cryoprotectant, or excipient, or the like.

The preservative, stabilizer or excipient may be comprised in the composition in an effective dose sufficient for reducing deterioration of the composition.

The term used in the present description, "deterioration" may include reduction of the number, reduction of activity or a combination thereof of the *Chlorella vulgaris* CD02-3002 strain comprised in the composition and the like.

For example, the composition may be one in which the *Chlorella vulgaris* CD02-3002 strain survives or remains active for a longer period of time in the composition, unlike the *Chlorella vulgaris* CD02-3002 strain present in nature, by comprising in an effective dose of preservative, stabilizer or excipient sufficient for reducing deterioration of the composition.

The preservative, stabilizer or excipient commonly used in the art may be used without limitation, as long as it can reduce deterioration of the composition. For example, the stabilizer may be any one or more selected from the group consisting of alginate, sodium alginate, casein, sodium casein, cellulose, methylcellulose, carboxymethylcellulose, sodium carboxymethylcellulose, pectin, gelatin, Arabia gum, xanthan gum, dextran, cyclodextrin and starch.

The cryoprotectant may be comprised in the composition in an effective dose sufficient for reducing deterioration of the composition, when the composition is freeze-dried. For example, unlike the *Chlorella vulgaris* CD02-3002 strain present in nature, the composition may be one in which the *Chlorella vulgaris* CD02-3002 strain survives or remains active for a longer period of time in the composition, by comprising in an effective dose of cryoprotectant sufficient for reducing deterioration of the composition in the freeze-dried state.

The cryoprotectant commonly used in the art may be used without limitation, as long as it can reduce deterioration of the composition in the freeze-dried state. For example, the cryoprotectant may be any one or more selected from the group consisting of glycerol, ethylene glycol, propylene glycol, dimethyl sulfoxide (DMSO), glucose, trehalose, maltodextrin, dextrin, skim milk and starch.

The composition may be added to a fertilizer of a plant by immersing, spraying or mixing.

Other aspect provides a fertilizer for plants comprising the composition for promoting plant growth.

The composition for promoting plant growth is as described above.

The plant may be a plant of the genus *Arabidopsis.* For example, the plant of the genus *Arabidopsis* may be *Arabidopsis thaliana,* but not limited thereto.

The fertilizer for plants may be prepared by a preparation method commonly used in the art. For example, the fertilizer for plants may further comprise an excipient or carrier commonly used in the art, and may be prepared in a formulation of a common fertilizer for plants known in the art.

The fertilizer for plants may be an eco-friendly fertilizer capable of replacing a chemical fertilizer.

The fertilizer for plants may be used for promoting plant growth.

Other aspect provides a method of culturing a *Chlorella vulgaris* CD02-3002 strain, comprising culturing a *Chlorella vulgaris* CD02-3002 strain having an effect of promoting plant growth, deposited with Accession number KCTC14659BP in the presence of fermentation exhaust gas.

The *Chlorella vulgaris* CD02-3002 strain is as described above.

The fermentation exhaust gas may comprise carbon dioxide of 0.5 to 20 %(v/v). For example, the fermentation exhaust gas may comprise carbon dioxide of 0.5 to 18%(v/v), 0.5 to 16%(v/v), 0.5 to 14%(v/v), 0.5 to 12%(v/v), 0.8 to 18%(v/v), 0.8 to 16%(v/v), 0.8 to 14%(v/v), or 0.8 to 12%(v/v).

The fermentation exhaust gas may be generated by microbial fermentation. For example, the fermentation exhaust gas may comprise carbon dioxide generated by microbial fermentation. The microorganism may include all microorganisms generating carbon dioxide during a fermentation or culturing process without limitation.

The culturing may be performed by supplying fermentation exhaust gas into a fermenter. For example, the fermentation exhaust gas may supply gas generated by microbial fermentation into a fermenter by filtering it. The fermentation exhaust gas may be supplied into a fermenter at a rate of 0.001 to 1 vvm. For example, the fermentation exhaust gas may be supplied into a fermenter at a rate of 0.001 to 1 vvm, 0.001 to 0.7 vvm, 0.001 to 0.5 vvm, 0.001 to 0.3 vvm, 0.001 to 0.2 vvm, 0.005 to 1 vvm, 0.005 to 0.7 vvm, 0.005 to 0.5 vvm, 0.005 to 0.3 vvm, 0.005 to 0.2 vvm, 0.01 to 1 vvm, 0.01 to 0.7 vvm, 0.01 to 0.5 vvm, 0.01 to 0.3 vvm, or 0.01 to 0.2 vvm.

The culturing may be performed at 20 to 40 °C. For example, the culturing may be performed at 20 to 30 °C.

The culturing may be performed in the presence of light. For example, the culturing may be performed while irradiating a sufficient amount of a light source for growing the *Chlorella vulgaris* CD02-3002 strain. The light source may be for example, an LED light source, but not limited thereto.

The culturing may be performed for 30 days or less. For example, the culturing may be performed for 3 to 30 days, 3 to 20 days, 3 to 15 days, 3 to 12 days, or 3 to 10 days.

The culturing may be performed by a known batch culturing method, continuous culturing method, fed-batch culturing method or the like, but not particularly limited thereto. Any medium and other culturing conditions used for culturing of the *Chlorella vulgaris* CD02-3002 strain of the present application may be used without particular limitation, as long as it is a medium used for culturing of a common *Chlorella vulgaris* strain, and for example, BG-11 medium may be used. Specifically, the *Chlorella vulgaris* CD02-3002 strain of the present application may be cultured by adjusting temperature, pH and the like in a common medium containing an appropriate carbon source, nitrogen source, phosphorus source, inorganic compound, amino acid and/or vitamin and the like. Specifically, proper pH may be adjusted using a basic compound (e.g.: sodium hydroxide, potassium hydroxide or ammonia) or acidic compound (e.g.: phosphoric acid or sulfuric acid), but not limited thereto.

Other aspect provides a method of promoting plant growth, comprising preparing a composition for promoting plant growth comprising any one or more selected from the group consisting of a *Chlorella vulgaris* CD02-3002 strain having an effect of promoting plant growth, deposited with Accession number KCTC14659BP; culture or culture supernatant of the strain; concentrate of the culture or culture supernatant; a dried matter of the culture, culture supernatant or concentrate; and lysate of the dried matter; and contacting the composition for promoting plant growth to a plant.

The *Chlorella vulgaris* CD02-3002 strain, and the composition for promoting plant growth are as described above.

The culture or culture supernatant of the *Chlorella vulgaris* CD02-3002 strain may be recovered using an appropriate method known in the art. In addition, the preparing a composition for promoting plant growth may use an appropriate method known in the art. For example, centrifugation, filtration, anion exchange chromatography, crystallization and HPLC and the like may be used, and a purification may be further comprised, and it may include a method of concentrating, drying and the like of microalgae or culture thereof known in the art.

The contacting may be performed by a method for treating the composition for promoting plant growth into soils, plants or seeds of plants. For example, the contacting may be performed by a method of spraying the composition for promoting plant growth to leaves of plants, but not limited thereto.

Other aspect provides a use for preparing a fertilizer for plants or a use for promoting plant growth, of a *Chlorella vulgaris* CD02-3002 strain having an effect of promoting plant growth, deposited with Accession number KCTC14659BP.

Other aspect provides a use for preparing a fertilizer for plants or a use for promoting plant growth, of a composition, comprising any one or more selected from the group consisting of a *Chlorella vulgaris* CD02-3002 strain having an effect of promoting plant growth, deposited with Accession number KCTC14659BP; culture or culture supernatant of the strain; concentrate of the culture or culture supernatant; a dried matter of the culture, culture supernatant or concentrate; and lysate of the dried matter.

### [ADVANTAGEOUS EFFECTS]

The novel *Chlorella vulgaris* CD02-3002 strain according to one aspect has a fast growth rate and high photosynthetic efficiency when cultured in the presence of fermentation exhaust gas, so exhaust gas due to microbial fermentation can be efficiently reduced, and when the culture or culture supernatant of the strain is treated on plants, there is an effect of promoting plant growth, and therefore, it can be used as a fertilizer for plants, and thus, it can be usefully used as a new carbon reduction technology that is environmentally friendly and has high industrial use.

### [BRIEF DESCRIPTION OF THE DRAWINGS]

FIG. 1 is a graph of the result of measuring the dry cell weight (DCW) after culturing each of 4 kinds of *Chlorella vulgaris* strains.
FIG. 2 is a graph of the result of measuring the aerial part (top part) weight of Arabidopsis thaliana, after treating the diluted solution of the culture supernatant of each of 4 kinds of DW, BG-11 liquid medium, Acadian 29 or *Chlorella vulgaris,* respectively.

### [MODE FOR INVENTION]

Hereinafter, the present invention will be described in more detail by examples. However, these examples are intended to illustratively describe one or more specific example, and the scope of the present invention is not limited by these examples.

### Example 1. Isolation of novel wild-type green algae

Samples were collected from a total of 5 river regions in Suwon, Gyeonggi-do, Korea, and microalgae belonging to green algae were isolated using a direct planting method.

Specifically, after collecting the samples, microalgae were isolated therefrom within 2~3 days, and the samples showing green color and comprising spherical morphology were smeared on BG-11 solid medium (1.5 g/L NaNO₃, 40 mg/L K₂HPO₄, 75 mg/L MgSO₄·7H₂O, 36 mg/L CaCl₂·2H₂O, 6 mg/L citric acid·H₂O, 6 mg/L ferric ammonium citrate, 1 mg/L Na₂EDTA·2H₂O, 20 mg/L Na₂CO₃, 2.86 mg/L H₃BO₃, 1.81 mg/L MnCl₂·4H₂O, 0.22 mg/L ZnSO₄·7H₂O, 0.39 mg/L Na₂MoO₄·2H₂O, 0.079 mg/L CuSO₄·5H₂O, 0.049 mg/L Co(NO₃)₂·6H₂O, 15 g/L agar) for microbial isolation. The obtained colonies were purely isolated through subculturing in the BG-11 solid medium inserted by adjusting the concentration of an antibiotic cocktail mix solution (0-500 mg/L Streptomycin sulfate, 0∼500 mg/L Ampicillin, 0∼500 mg/L Kanamycin sulfate).

### Example 2. Identification of DNA sequence of isolated microalgae

For molecular biological identification of the microalgae isolated in Example 1 above, the 18s rRNA sequence was analyzed.

Specifically, after extracting DNA from the purely isolated colonies, using primers for amplifying a gene of the 18s rRNA region (Table 1), PCR was carried out.

**[Table 1]**

| **SEQ ID NO:** | **Primer** | **Sequence (5' -> 3')** |
|---|---|---|
| **2** | 18s-F | CGACTTCTGGAAGGGACGTA |
| **3** | 18s-R | CTAGGTGGGAGGGTTTAATG |

PCR reaction was performed by denaturation at 95°C for 5 minutes, and then repeating denaturation at 95°C for 30 seconds, annealing at 55°C for 30 seconds and polymerization at 72°C for 2 minutes 35 times, and then polymerization at 72°C for 5 minutes. As the result of analyzing the nucleotide sequence utilizing the amplified reaction solution, a nucleotide sequence in an about 1,565 bp size was secured (SEQ ID NO: 1). As the result of BLAST search of this on NCBI (https://www.ncbi.nlm.nih.gov/), it was confirmed that it had homology of 99.68% to the *Chlorella vulgaris* KMMCC FC-42 strain, and the isolated strain was named *Chlorella vulgaris* CD02-3002, and was deposited to Korea Research Institute of Bioscience and Biotechnology, Korean Collection for Type Cultures (KCTC), an international depository under the Budapest Treaty, on August 23, 2021, and was given Accession number KCTC14659BP.

### Example 3. Confirmation of growth ability of Chlorella vulgaris CD02-3002 strain under light cultivation condition based on fermentation exhaust gas

In order to confirm whether the isolated novel strain *Chlorella vulgaris* CD02-3002 is a strain capable of growing under a condition of light cultivation based on fermentation exhaust gas, the growth ability was compared and evaluated to various kinds of *Chlorella vulgaris* strains.

As a control strain, *Chlorella vulgaris* UTEX265 strain received from UTEX Culture Collection of Algae at UT-Austin institution was used, and as a comparative group, the *Chlorella vulgaris* CD11-2001 and CD11-2003 strains isolated by the same method as described in Example 1 were used. Each strain was inoculated in a sterilized BG-11 liquid medium (1.5 g/L NaNO₃, 40 mg/L K₂HPO₄, 75 mg/L MgSO₄·7H₂O, 36 mg/L CaCl₂·2H₂O, 6 mg/L citric acid·H₂O, 6 mg/L ferric ammonium citrate, 1 mg/L Na₂EDTA·2H₂O, 20 mg/L Na₂CO₃, 2.86 mg/L H₃BO₃, 1.81 mg/L MnCl₂·4H₂O, 0.22 mg/L ZnSO₄·7H₂O, 0.39 mg/L Na₂MoO₄·2H₂O, 0.079 mg/L CuSO₄·5H₂O, 0.049 mg/L Co(NO₃)₂·6H₂O) 100 mL, and then was cultured for 4~7 days while light was continuously irradiated with an LED light source, and CO₂ at a concentration of 1~10%(v/v) was supplied by 0.01-0.1 vvm. After that, the culturing solution was inoculated to a new sterilized BG-11 liquid medium 900 mL, and was cultured for 3∼5 days while light was continuously irradiated with an LED light source, and during the culturing period, fermentation exhaust gas was directly supplied to the culturing solution by 0.01-0.5 vvm after passing through an air filter. As the fermentation exhaust gas, gas generated during fed-batch culturing Escherichia coli CJ181 (KFCC 10902) strain (U.S. Patent Publication US 2008/0299644 A1) in a fermenter while maintaining the culturing temperature of 30°C, culturing pH 6.9-7.1 (adjusting pH with ammonia water), aeration amount 0.5-1.0 vvm and stirring rate of 500∼700 rpm was used.

After completion of the culturing, each strain culturing solution 10 mL was filtered with a 0.45 µm filter, and then dried in a 60°C oven for 24 hours, and then, by calculating a difference of the weight of the filter before filtration, and the weight of the filter comprising dried cells after drying, a dry cell weight (DCW) value was calculated.

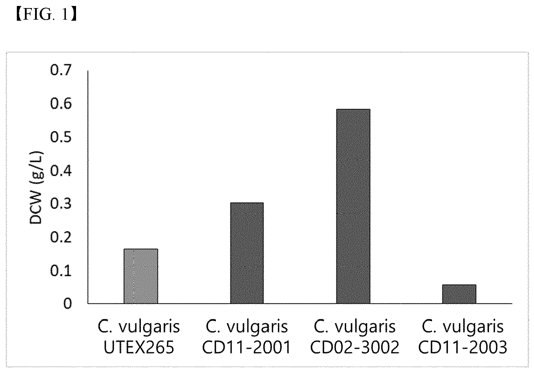

As a result, as shown in FIG. 1, it was confirmed that the *Chlorella vulgaris* CD02-3002 strain had a significantly high dry cell weight compared to the received *Chlorella vulgaris* strain (UTEX265) or novel isolated other *Chlorella vulgaris* strains (CD11-2001 and CD11-2003). Through this, it could be found that the *Chlorella vulgaris* CD02-3002 strain was a strain with the most excellent growing rate under the condition of light cultivation based on fermentation exhaust gas compared to other *Chlorella vulgaris* strains.

### Example 4. Confirmation of effect of promoting plant growth of culture supernatant of Chlorella vulgaris CD02-3002 strain

In order to confirm the effect of promoting plant growth of the culture supernatant of the *Chlorella vulgaris* CD02-3002 strain, promoting growth was evaluated for *Arabidopsis thaliana.*

Specifically, wild-type *Arabidopsis thaliana* seeds were prepared by surface sterilizing in 70% ethanol for 1 minutes and 1% NaOCl solution for 3 minutes, and then washing with sterilized distilled water. The *Arabidopsis thaliana* was refrigerated (4°C) for 2 days and then was planted in the top soil (Dongbu Farm Hannong Co., Ltd.) and then was cultivated in a 21°C thermostat for about 1 week in a dark state in which light is irradiated for 12 hours and light is not irradiated for 12 hours.

A total 4 kinds of *Chlorella vulgaris* UTEX265, CD11-2001, CD02-3002, and CD11-2003 were inoculated into sterilized BG-11 liquid medium 200 mL, and then were cultured at 25°C for 5~7 days while an LED light source was irradiated and 1~10%(v/v) CO₂ was continuously supplied by 0.1 vvm. The cultured seed culture was inoculated into sterilized BG-11 liquid medium 1.8 L and was cultured under the same condition for 10 days at maximum. After completion of the culturing, the culture was centrifuged to obtain the culture supernatant of each strain.

In order to evaluate the effect of promoting plant growth, the cultivated *Arabidopsis thaliana* was treated with a 10~1000-fold dilution of the culture supernatant of each strain at a level of 1mL/plant, and foliar fertilization was applied three times at an interval of 4 days. As a control group, a group in which distilled water (DW) was equally treated was set, and as a comparative group, a group in which a medium used for microalgae light cultivation (BG-11 liquid medium) or commercialized seaweed extract liquid fertilizer (Acadian 29) were equally treated was added. The aerial part live weight (aerial part weight) was measured as a growth measurement index of *Arabidopsis thaliana.* The mean value and standard deviation of the top part live weight were calculated and shown by performing repeated experiments with *Arabidopsis thaliana* 8 subjects for each test group.

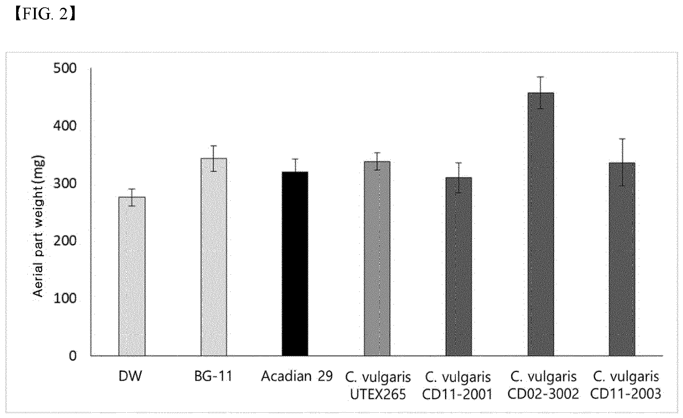

As a result, as shown in FIG. 2, it was shown that other *Chlorella vulgaris* strains had little or no effect of promoting growth compared to the control group, while the *Chlorella vulgaris* CD02-3002 strain showed a significantly excellent effect of promoting growth, and it could be confirmed that this was a significantly excellent effect than a commercialized fertilizer, Acadian 29.

From the above description, those skilled in the art to which the present application pertains will be able to understand that the present application may be embodied in other specific forms without changing the technical spirit or essential characteristics. In this regard, it should be understood that the examples described above are illustrative and not restrictive in all respects. The scope of the present application should be construed as including all changes or modifications derived from the meaning and scope of the claims to be described below and equivalent concepts rather than the detailed description above, in the scope of the present application.

### [ACCESSION NUMBER]

Depository authority name: Korea Research Institute of Bioscience and Biotechnology, Korean Collection for Type Cultures (KCTC)
Accession number: KCTC14659BP
Accession date: 20210823

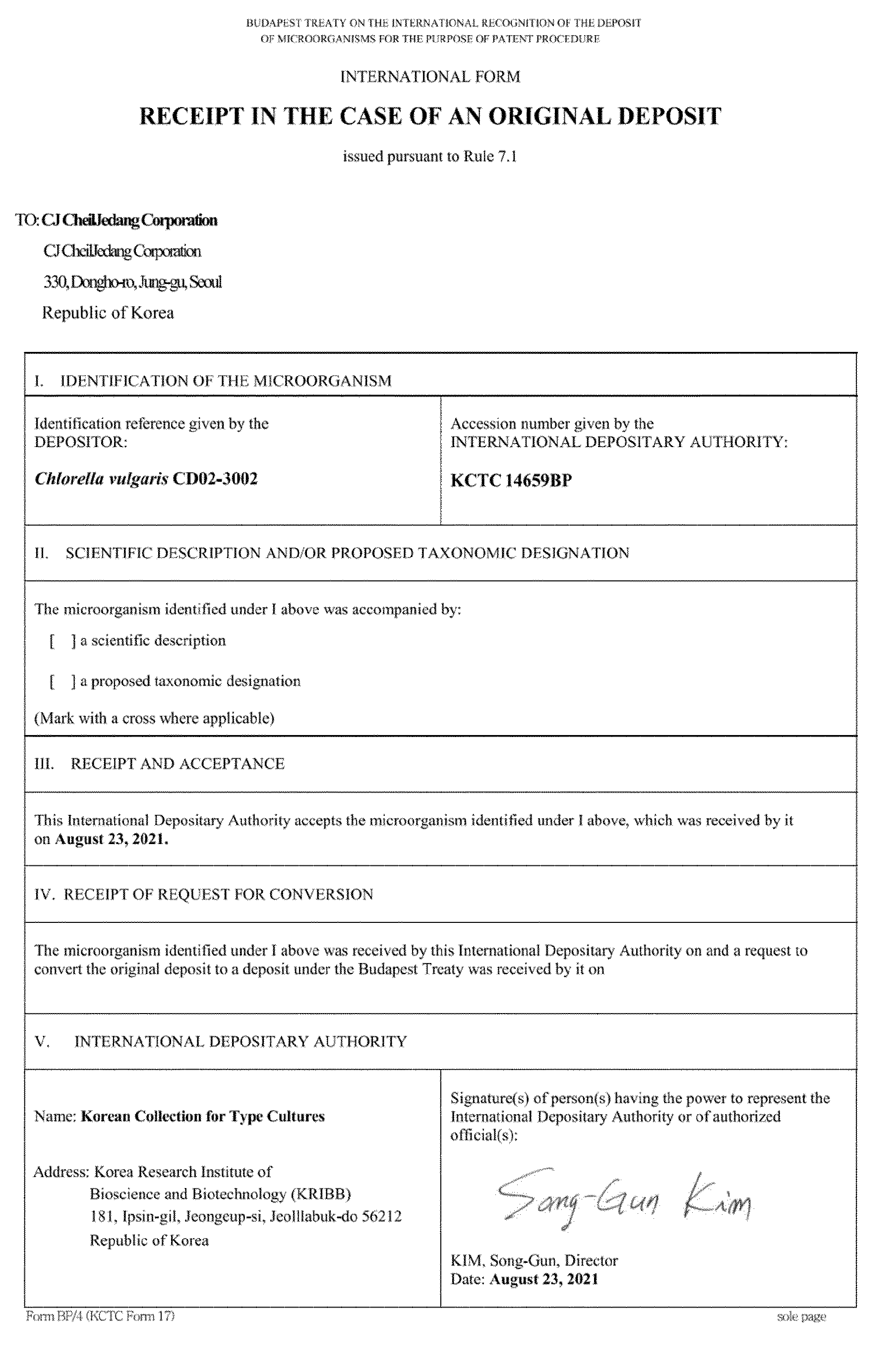
BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO: CJ CheilJedang Corporation
CJ CheilJedang Corporation
330, Dongho-ro, Jung-gu, Seoul
Republic of Korea

I. IDENTIFICATION OF THE MICROORGANISM
Identification reference given by the
DEPOSITOR: | Accession number given by the
INTERNATIONAL DEPOSITARY AUTHORITY:
Chlorella vulgaris CD02-3002 | KCTC 14659BP
II. SCIENTIFIC DESCRIPTION AND/OR PROPOSED TAXONOMIC DESIGNATION
The microorganism identified under I above was accompanied by:
[ ] a scientific description
[ ] a proposed taxonomic designation
(Mark with a cross where applicable)
III. RECEIPT AND ACCEPTANCE
This International Depositary Authority accepts the microorganism identified under I above, which was received by it
on August 23, 2021.
IV. RECEIPT OF REQUEST FOR CONVERSION
The microorganism identified under I above was received by this International Depositary Authority on and a request to
convert the original deposit to a deposit under the Budapest Treaty was received by it on
V. INTERNATIONAL DEPOSITARY AUTHORITY
Name: Korean Collection for Type Cultures

Address: Korea Research Institute of
Bioscience and Biotechnology (KRIBB)
181, Ipsin-gil, Jeongeup-si, Jeolllabuk-do 56212
Republic of Korea | Signature(s) of person(s) having the power to represent the
International Depositary Authority or of authorized
official(s):
KIM, Song-Gun, Director
Date: August 23, 2021
Form BP/4 (KCTC Form 17) sole page

## Claims

1. A *Chlorella vulgaris* CD02-3002 strain having an effect of promoting plant growth, deposited with Accession number KCTC14659BP.

2. A composition for promoting plant growth, comprising any one or more selected from the group consisting of a *Chlorella vulgaris* CD02-3002 strain having an effect of promoting plant growth, deposited with Accession number KCTC14659BP; culture or culture supernatant of the strain; concentrate of the culture or culture supernatant; a dried matter of the culture, culture supernatant or concentrate; and lysate of the dried matter.

3. A fertilizer for plants comprising the composition for promoting plant growth of Claim 2.

4. A method of culturing a *Chlorella vulgaris* CD02-3002 strain, comprising culturing a *Chlorella vulgaris* CD02-3002 strain having an effect of promoting plant growth, deposited with Accession number KCTC14659BP in the presence of fermentation exhaust gas.

5. The method of culturing a *Chlorella vulgaris* CD02-3002 strain according to claim 4, wherein the fermentation exhaust gas comprises carbon dioxide of 0.5 to 20 %(v/v).

6. The method of culturing a *Chlorella vulgaris* CD02-3002 strain according to claim 4, wherein the fermentation exhaust gas is generated by microbial fermentation.

7. The method of culturing a *Chlorella vulgaris* CD02-3002 strain according to claim 4, wherein the culturing is performed at 20 to 40 °C.

8. The method of culturing a *Chlorella vulgaris* CD02-3002 strain according to claim 4, wherein the culturing is performed for 30 days or less.

9. A method of promoting plant growth, comprising
preparing a composition for promoting plant growth comprising any one or more selected from the group consisting of a *Chlorella vulgaris* CD02-3002 strain having an effect of promoting plant growth, deposited with Accession number KCTC14659BP; culture or culture supernatant of the strain; concentrate of the culture or culture supernatant; a dried matter of the culture, culture supernatant or concentrate; and lysate of the dried matter; and
contacting the composition for promoting plant growth to a plant.

10. The method according to claim 9, wherein the contacting is performed by a method of treating the composition for promoting plant growth to soil, a plant or a seed of a plant.
